# EUROPEAN PATENT APPLICATION

(11) **EP 1 384 784 A1**
(43) Date of publication of application: **28.01.2004**
(21) Application number: 02720014.6
(22) Date of filing: 01.04.2002
(51) Int. Cl.: C12N 15/63, C12N 9/00, C07H 21/02, A61K 48/00, A61K 31/7105

(54) **METHOD FOR REVERSIBLE INHIBITION OF GENE EXPRESSION BY MODIFIED RIBONUCLEOPROTEINS**

(30) Priority: 30.03.2001 ES 200100765
(71) Applicant: INSTITUTO CIENTIFICO Y TECNOLOGICO DE NAVARRA, S.A., 31008 Pamplona (ES)
(72) Inventor: FORTES ALONSO, Purificación, E-31008 Pamplona (ES); PRIETO VALTUENA, Jesús, E-31008 Pamplona (ES); IAN GUNDERSON, Samuel, E-31008 Pamplona (ES)
(74) Representative: Elzaburu, Alberto de
(86) International application number: PCT/ES2002/000162
(87) International publication number: WO 2002/083908

(57) **Abstract**

The invention modifies the U1 ribonucleoprotein (U1 snRNP) at the 5' end of its RNA so that it binds specifically to an mRNA of the gene to be inactivated, in such a way that its expression is inhibited. Gene inactivation is greater with the binding of several ribonucleoproteins to several binding sites on the 3' terminal exon of the mRNA of the gene to be inactivated, concretely at specific sites around the site of initiation of polyadenylation of said mRNA. This method is used for (i) inhibiting the expression of genes of unknown function so that they can be studied and (ii) inhibiting the expression of genes that are harmful to the cell.

## Description

### Technical field of the invention

The invention relates to the field of the inactivation of genes whose expression is either unknown, or is harmful for the cell where expression occurs. It also relates to ribonucleoproteins, proteins that are able to inhibit gene expression.

### STATE OF THE ART

Effective and specific inhibition of gene expression is the basis of some protocols of gene therapy and of functional studies of genes. The expression of certain genes sometimes proves toxic for the cell, causing diseases, such as the expression of viral genes in infected cells, dominant negatives in hereditary diseases, or genes whose expression should be restricted, such as oncogenes. Inhibition of expression of these toxic genes would make it possible to cure the diseases that they trigger. Moreover, studies in genomics and proteomics enable a large number of genes to be isolated, some of them of unknown function. These techniques make it possible to classify these genes according to their levels of expression or according to the circumstances that permit it, but they do not say much about their function. Systematic and specific inhibition of the expression of these genes would enable us to develop simple protocols for determining their function.

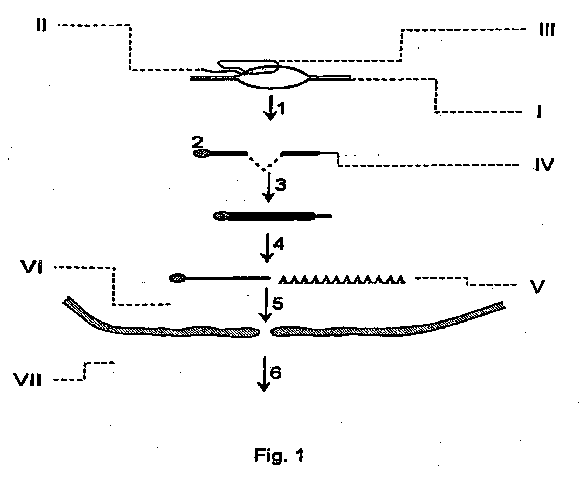

The simplest reversible form of specific inhibition of the expression of a gene is to lower the stability of the RNA that is transcribed by it or block its translation to protein. The systems used most, based on antisense technology (antisense ribozymes and oligos), have not had much success, perhaps because in order to function they have to interact with target RNAs in vivo, and these are usually covered with proteins and form secondary or tertiary structures that protect them from these interactions. A possible alternative is to utilize the functional properties of small nuclear ribonucleoproteins (snRNPs), which interact with specific sequences of the pre-mRNAs and participate in various stages of the mRNA maturation process in the nucleus (Fig. 1). In the case of the U1 snRNP, the 5' end of its RNA (U1 snRNA) hybridizes by base-pairing with the 5' end of the sequences that are to be eliminated, the introns, in the cutting and splicing of the pre-mRNA. This interaction is stabilized by proteins that interact directly or indirectly with U1 snRNA and with the mRNA that is to be processed.

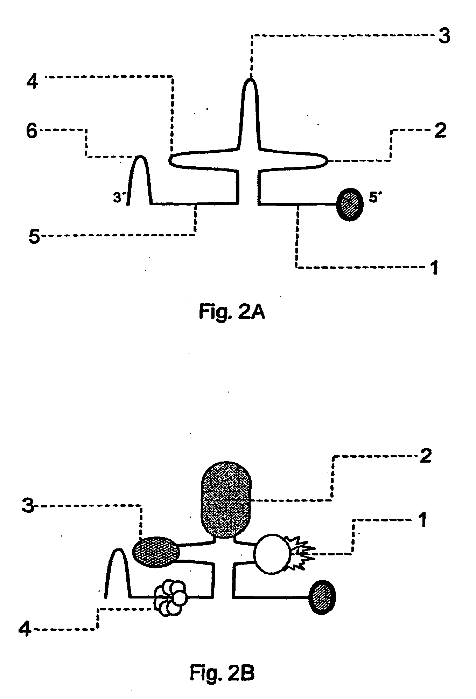

U1 snRNA (Fig. 2A) is an RNA transcribed by RNA polymerase II that undergoes a complex process of maturation. After its transcription in the nucleus it is quickly transported to the cytoplasm. There, interaction with Sm proteins and hypermethylation of its 5' end permit it to be carried back to the nucleus, where it accumulates first in the Cajal bodies and perhaps in the nucleolus, where it seems to be modified by pseudouridinylation. Once the U1 snRNP is mature, it accumulates in interchromatin granules or "speckles" where it is active in the processing of mRNA. Moreover, U1 snRNP forms a stable bond with proteins U1A, 70K and U1C (Fig. 2B). These, together with the Sm proteins, increase the efficiency with which U1 snRNA recognizes the pre-mRNA.

Once U1 snRNP interacts with the pre-mRNA, a series of bondings of other proteins and snRNPs is triggered, which then cause elimination of the intron. For this to be possible, it is necessary for U1 snRNP to separate from the pre-mRNA.

To ensure correct processing of introns, U1 snRNP, on binding to the RNA, inhibits the action of the machinery of rupture and polyadenylation on adjacent sequences. The 70K protein of U1 snRNP is able to bond to the carboxyterminal end of the poly(A) polymerase (PAP) subunit of the polyadenylation complex, inhibiting its function (Gunderson et al., 1997, 1998). This generalized cellular mechanism is also employed for controlling the expression of some cellular genes, such as the mRNA of U1A or of some viral RNAs, as occurs in the human immunodeficiency virus (HIV) or the bovine papilloma virus (BPV). An excess of U1A, another U1 snRNP protein, can inhibit the polyadenylation of the messenger that codes for it (Boelens et al., 1993). The mechanism has been described in detail. The 3' end of the mRNA of U1A has a particular structure to which two molecules of U1A bind (van Gelder et al., 1993). The zone of interaction of these two molecules acts as an effector domain, as it mimics the 70K domain that is able to bond to the terminal end of the PAP and thus inhibit its function (Gunderson et al., 1994; 1998; Klein Gunnewiek et al., 2000). The mechanism employed by HIV and BPV is different and has received less study. The 5' end of the intron is upstream of the sequences of rupture and polyadenylation of the RNA of BPV (Furth et al., 1991; 1994) and downstream in the 5' LTR of HIV (Ashe et al., 1995; 1997). Probably on account of this different localization, U1 snRNP inhibits the polyadenylation of the RNA of BPV and rupture of the RNA of HIV. It is not known which molecule(s) of U1 snRNP is (are) involved in this inhibition of rupture because, in contrast to what occurs for the inhibition of polyadenylation, the complete U1 snRNP is required, not just 70K. It has been possible to reproduce this phenomenon in tests of rupture and polyadenylation in vitro and with partially purified systems (Vagner et al., 2000).

It would be interesting to use the inhibition that U1 snRNP exerts on the machinery of rupture and polyadenylation to control gene expression. If molecules of U1 snRNA whose 5' end has been modified so that it recognizes sequences adjacent to polyadenylation sequences in an actual messenger are expressed in the cell, it would be possible to inhibit the polyadenylation of this messenger specifically. U1 snRNPs modified at their 5' end have been used in vivo to study their effect on the process of cutting and splicing, but no modifications of U1 snRNP for inhibiting the machinery of polyadenylation have been described. This approximation of modification of U1 snRNP for altering the process of cutting and splicing has been used for correcting defects in the processing of genes involved in genetic diseases such as beta-thalassemia (Hitomi et al., 1998; Gorman et al., 2000). Another snRNP called U7 has also been used successfully for correcting processing defects involved in beta-thalassemia (Gorman et al., 1998; Suter et al., 1999).

In addition to inhibition of polyadenylation, there are three other mechanisms by which a modified U1 snRNP could specifically inhibit the expression of a gene with which mRNA interacts: i) by acting as antisense, ii) by inhibiting its transport to the cytoplasm and, if this interaction occurs at the non-coding 3' end of the mRNA, ii) by activating its decay by the mechanism of decay mediated by nonsense mutations or "nonsense mediated decay" (reviewed by Lykke-Andersen, 2001).

There are examples in the literature of the use of U1 snRNAs modified so that they act as antisense or so that they include sequences of ribozymes. The main advantages of including these sequences in U1 snRNP are that it ensures their stability and their localization in the cell nucleus where it is hoped the effect will be greater. tRNAs and U6 snRNAs have also been modified to include ribozymes in their sequences with the same advantages (Biasolo et al., 1996; Good et al., 1997; Zhao and Lemke, 1998; Kuwabara et al., 1998; Medina and Joshi, 1999). In the case of U1 snRNP, only its promoter and terminator sequences have been used for directing the expression of ribozymes (Bertrand et al., 1997), or the DNA of the ribozyme has been introduced in place of the sequences of U1 snRNP that enable it to interact with U1C (Michienzi et al., 1996) or with the Sm proteins (Montgomery and Dietz, 1997; Michienzi et al., 1998). Antisense sequences without ribozyme activity have also been introduced in place of the sequences for binding to Sm proteins (Liu, 1997).

There has been no description of the use of modified U1 snRNPs that control gene expression by altering polyadenylation or the transport of the target RNA to the cytoplasm nor that activate its nonsense mediated decay. In the first case and in the last case, these U1 snRNPs would have to be directed against the noncoding 3' region, whereas for inhibiting mRNA transport or for acting as antisense, the U1 snRNPs could be directed against any region of the mRNA.

In the antisense cases described, using modified U1 snRNPs, inhibitions of gene expression from 50 to 95%, relative to expression without inhibition, have been achieved, corresponding to an inhibition ratio (expression without inhibition / expression with inhibition) of 2 to 20 times.

By directing the modified U1 snRNPs against the 3' terminal exon or the noncoding 3' region of a target mRNA, more routes of decay could be activated, and greater inhibitions could be attained than those described to date.

### BIBLIOGRAPHY

Ashe, M. P., Griffin, P., James, W., Proudfoot, N. J. (1995). Poly(A) site selection in the HIV-1 provirus: inhibition of promoter-proximal 1 polyadenylation by the downstream major splice donor site. Genes & Dev. 9, 3008-3025.
Ashe, M. P., Pearson, L. H. y Proudfoot, N. J. (1997). The HIV-1 5' LTR poly(A) site is inactivated by U1snRNP interaction with the downstream major splice donor site. Embo J. 16, 5752-5763.
Bertrand, E., Castanotto, D., Zhou, XC., Carbonnelle, C., Lee, N. S., Good, P., Chatterjee, S., Grange, T., Pictet, R., Kohn, D., Engelke, D. Y Rossi, J. J. (1997). The expression cassette determines the functional activity of ribozymes in mammalian cells by controlling their intracellular localization. RNA 3, 75-88.
Biasolo, M. A., Radaelli, A., Pup, L., Franchin, E., Giuli-Morghen, C. y Palu, G. (1996). A new antisense tRNA construct for the genetic treatment of human immunodeficiency virus type I infection. J. Virol. 70, 2154-2161.
Boelens, W. C., Jansen, E. J. R., van Venrooij, W. J., Stripecke, R., Mattaj, I. W. y Gunderson, S. I. (1993). The human U1 snRNP-specific U1A protein inhibits polyadenilacion of its own pre-mRNA. Cell 72, 881-892.
Cohen, J. B., Broz, S. D. y Levinson, A. D. (1993). U1 small nuclear RNAs with altered specificity can be stably expressed in mammalian cells and promote permanent changes in pre-mRNA splicing. Mol. Cell. Biol. 13, 2666-2676.
Cohen, J. B., Snow, J. E., Spencer, S. D. Y Levinson A. D. (1994). Suppression of mammalian 5' splice-site defects by U1 small nuclear RNAs from a distance. Proc. Natl. Acad. Sci. USA 91, 10470- 10474.
Furth, P. A. y Baker, C. C. (1991). An element in the bovine papillomavirus late 3' untranslated region reduces polyadenylated cytoplasmic RNA levels. J. Virol. 65, 5806-5812.
Furth, P. A., Choe, W. T., Rex, J. H., Byrne, J. C. y Baker, C. C. (1994). Sequences homologous to 5' splice sites are required for the inhibotory activity of papillumavirus late 3' untranslated regions. Mol. Cell. Biol. 14, 5278-5289.
Good, P. D., Krikos, A. J., Li, S. X. L., Bertrand, E., Lee, N. S., Giver, L., Ellington, A., Zaia., J. A., Rossi, J. J. Y Engelke, D. R. (1997). Expression of small, therapeutic RNAs in human cell nuclei. Gene therapy 4, 45-54.
Gorman, L., Suter, D., Emerik, V., Schumperli, D. y Kole R. (1998). Stable alteration of pre-mRNA splicing patterns by modified U7 small nuclear RNAs. Proc. Natl. Acad. Sci. USA 95, 4929-4934.
Gorman, L., Mercatante, D. R. y Kole R. (2000). Restoration of correct splicing of thalassemic beta-globin pre-mRNA by modified U1 snRNAs. JBC 275, 35914-35919.
Gunderson, S. I., Beyer, K., Martin, G., Keller, W., Boelens, W. C. y Mattaj, I. W. (1994). The human U1A snRNP protein regulates polyadenylation via a direct interaction with poly(A) polymerase. Cell 76, 531-541.
Gunderson, S. I., Vagner, S., Polycarpou-Schwarz, M. y Mattaj, I. W. (1997). Involvement of the carboxyl-terminus of vertebrate poly(A) polymerase in U1A autoregulation and in the coupling of splicing and polyadenylation. Genes & Dev. 11, 761-773.
Gunderson, S. I., Polycarpou-Schwarz, M. y Mattaj, I. W. (1998). U1 snRNP inhibits pre-mRNA polyadenylation through a direct interaction between U1 70K and poly(A) polymerase. Mol. Cell 1, 255-264.
Hitomi, Y., Sugiyama, K. Y Esumi, H. (1998). Suppression of the 5' splice site mutation in the Nagase analbuminemic rat with mutated U1 snRNA. Biochem Biophys Res Commun 251, 11-16.
Klein Gunnewiek, J. M., Hussein, R. I., van Aarssen, Y., Palacios, D., de Jong, R., van Venrooij, W. J. y Gunderson S. I. (2000). Fourteen residues of the U1 snRNP-specific U1A protein are required for homodimerization, cooperative RNA binding, and inhibition of polyadenylation. Mol Cell Biol. 20, 2209-2217.
Kuwabara, T., Warashina, M., Orita, M.,Koseki, S., Ohkawa, J. y Taira, K. (1998). Formation of a catalytically active dimer by tRNA(Val)-driven short ribozymes. Nat. Biotechnol 16, 961-965.
Lund, E. y Dahlberg, J. E. (1984). True genes for human U1 small nuclear RNA. Copy number, polymorphism and methylation. J. Biol. Chem 259, 2013-2021.
Lund, E. (1988). Heterogeneity of human U1 snRNAs. Nucleic Acids Res 16, 5813-5826.
Lykke-Andersen, J. (2001). MRNA quality control: Marking the message for life or death. Current Biol. 11, R88-R91.
Liu D., Donegan, J., Nuovo, G., Mitra, D. Y Laurence, J. (1997). Stable human immunodeficiency virus type I (HIV-I) resistance in transformed CD4+ monocytic cells treated with multitargeting HIV-I antisense sequences incorporated into U1 snRNA. J. Virol. 71, 4079-4085.
Medina, M. F. y Joshi, S. (1999). Design, characterization and testing of tRNA3Lys-based hammerhead ribozymes. Nucleic Acids Res. 27, 1698-1708.
Michienzi, A., Prislei, S. Y Bozzoni I. (1996). U1 small nuclear RNA chimeric ribozymes with substrate specificity for the Rev pre-mRNA of human immunodeficiency virus. Proc. Natl. Acad. Sci. USA 93, 7219-7224.
Michienzi, A., Conti, L., Varano, B., Prislei, S., Gessani y Bozzoni, I. (1998). Inhibition of human immunodeficiency virus type I replication by nuclear chimeric anti-HIV ribozymes in a human T lymphoblastoid cell line. Human gene therapy 9: 621-628.
Montgomery, R. A. y Dietz, H. C. (1997). Inhibition of fibrillin 1 expression using U1 snRNA as a vehicle for the presentation of antisense targeting sequence. Human Molecular Genetics 6, 519-525.
Suter, D., Tomasini, R., Reber, U., Gorman, L., Kole, R. y Schumperli, D. (1999). Double-target antisense U7 snRNAs promote efficient skipping of an aberrant exon in three human beta-thalassemic mutations. Hum. Mol. Genet. 8, 2415-2423.
Vagner S., Ruegsegger, U., Gunderson, S. I., Keller, W. y Mattaj, I. W. (2000). Position dependent inhibition of the cleavage step of pre-mRNA 3' end processing by U1 snRNP. RNA. 6, 178-188.
van Gelder C. W., Gunderson, S. I., Jansen, E. J., Boelens, W. C., Polycarpou-Scharz, M., Mattaj, I. W. y va Venrooij W. J. (1993). A complex secondary structure in U1A pre-mRNA that binds two molecules of U1A protein is required for regulation of polyadenilation. Embo J. 15, 5191-5200.
Watanabe, N. y Ohshima, Y. (1988). Three types of rat U1 small nuclear RNA genes with different flanking sequences are induced to express in vivo. Eur J Biochem 174, 125-132.
Zhao, J. J. Y Lemke, G. (1998). Selective disruption of neuregulin-1 function in vertebrate embryos using ribozyme-tRNA transgenes. Development 125, 1899-1907.

### DESCRIPTION OF THE INVENTION

The present invention relates to U1 snRNPs that have been modified for reversibly inhibiting the expression of a target gene, characterized in that said U1 snRNPs can hybridize with a sequence contained within the 3' terminal exon of the messenger RNA of the target gene. Thus, in the present invention it is claimed that when the modified U1 snRNPs hybridize with the mRNA, they act upon the machinery of polyadenylation, and for this they must hybridize relatively near to where this machinery operates.

Preferably, the U1 snRNPs of the present invention have been modified at their 5' end, which has a natural capacity to interact with the messenger RNA, so that the rest of the structure of the U1 snRNP, and hence its localization, its stability and its activity will not be compromised. More preferably, the modifications have been effected in at least one nucleotide included within the first 15 nucleotides of the 5' terminal end of the modified ribonucleoprotein.

In a preferred embodiment of the present invention, the U1 snRNPs have been modified to permit hybridization with a sequence contained within the 3' terminal exon larger than 7 nucleotides, more preferably between 7 and 15.

The U1 snRNPs of the present invention can act by inhibiting the transport of the target mRNAs to the cytoplasm. The messengers with introns are normally retained in the cell nucleus until they are processed. However, if U1 snRNP interacts with the messenger, the transport machinery could identify it as unprocessed and hence it would accumulate in the nucleus where it could be degraded. The same U1 snRNPs of the present invention could act by activating the "nonsense mediated decay" mechanism, mediated by interaction with the noncoding RNA sequences, which would initiate a cascade of protein interactions that establish a cryptic 3' site of cutting and splicing in the mRNA, ending in the elimination of a fictitious intron. For this to occur, it is necessary for the interaction of U1 snRNP to take place at more than 50 nucleotides downstream of the stop codon, i.e. in the noncoding 3' region. These routes of action will also help to achieve a greater inhibitory capacity of the U1 snRNP modified ribonucleoproteins of the present invention.

### 1. Modification of the 3' region corresponding to the 3' terminal exon of reporter genes so that they interact with the cellular U1 snRNP.

### A. Introduction of a site for binding to U1 snRNP

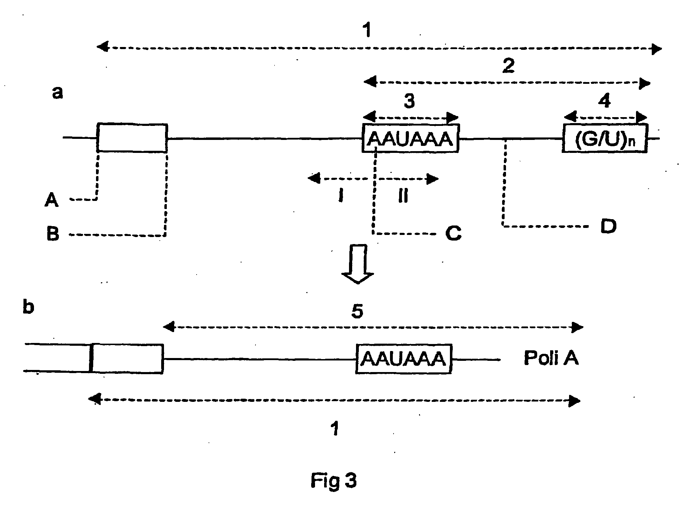

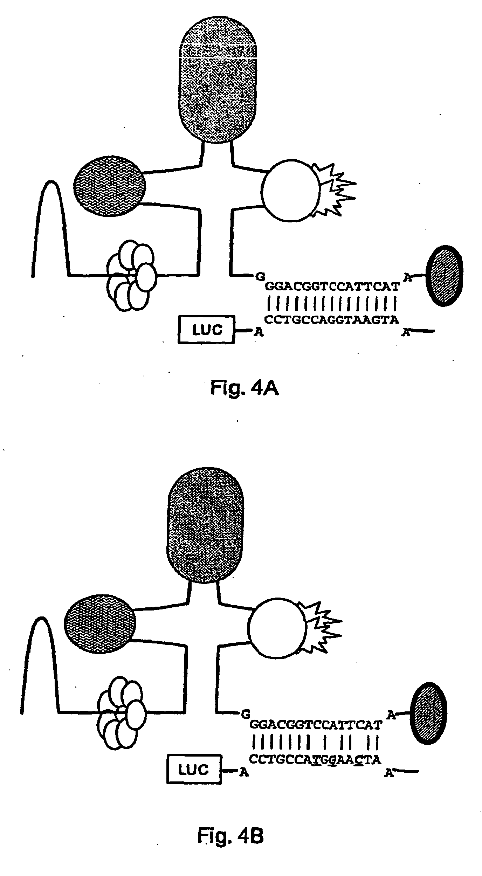

To find out which mRNA sequences are most susceptible to the action of U1 snRNP, distinct sequences recognizable by endogenous U1 snRNP were introduced in the 3' terminal exon of the mRNA of reporter genes with distinct polyadenylation sequences. The reporter genes used are those of firefly luciferase (LUC) and renilla luciferase (RL) with SV40 polyadenylation sequences. In another construct a synthetic polyadenylation site was incorporated in the renilla reporter near its translation terminating sequence. The sequences for binding to the U1 snRNPs could be downstream or upstream of the first adenine of the polyadenylation sequences (Fig. 3). In this concrete case the binding sequences were introduced upstream. To indicate their location, the nucleotide corresponding to the first adenine of the first consensus sequence of polyadenylation is used as nucleotide 0, counting upstream from there (as far as the 5' end with negative numbers). The U1 snRNP binding sequences were placed in positions -145 (constructs LUC-145 and RL-145) and -87 (constructs LUC-87 and RL-87) of the messenger of LUC and RL with SV40 polyadenylation sequences. In the case of RL with the synthetic polyadenylation sequence, modification was effected at position -24 (construct RL-24). All these U1 snRNP binding sites are in the 3' terminal exon. In all cases, sequences with three point mutations (Fig. 4B), sequences that we shall refer to as LucM and RLM respectively, were used as negative controls of binding to endogenous U1 snRNP (Fig. 4A).

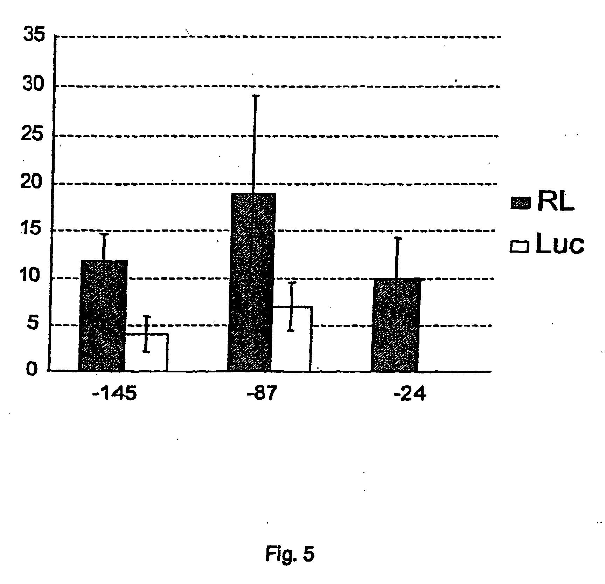

These constructions were used for transfecting HeLa cells. When transfecting the modified luciferase constructs unmodified renilla was used, and vice versa, as transfection control. All the data are corrected with respect to the same efficiency of transfection, showing the mean of three measurements per experiment and a minimum of three experiments for each case. Fig. 5 shows the results expressed as inhibition ratio: activity without inhibition (i.e. obtained with LucM) over activity with inhibition (i.e. obtained with Luc). The results show an inhibition ratio of 4 to 7 times for the luciferase messenger and 10 to 20 times for the renilla messenger. These inhibitions are at the level of that described using U1 snRNPs modified with antisense sequences. Similar results were obtained using other cell types such as COS-1 and BHK. Moreover, greater inhibition is obtained in both reporters with U1 snRNP directed against the -87 position (7 and 20 times) than against the -145 position (4 and 12 times) therefore some positions in the messenger could be more prone to the action of U1 snRNP. Finally, the effect of U1 snRNP is greater in the messenger of renilla (12 to 20 times) than in the messenger of luciferase (from 4 to 7 times in the same positions). This may be due to the fact that usually 8 times less plasmid of renilla than of luciferase was transfected, since its activity is three logarithms greater. To analyze whether the effect of U1 snRNP is dependent on the mRNA dose, we analyzed inhibition by transfecting increasing doses of plasmid. Inhibition was greater with the minimum dose, indicating that the mechanism is more efficient with less abundant RNAs. Inhibition occurs independently of the type of 3' terminal exon used, as there is inhibition of the messenger of renilla both when it has sequences of SV40 or when it has synthetic sequences.

### B. Introduction of various U1 snRNP binding sites

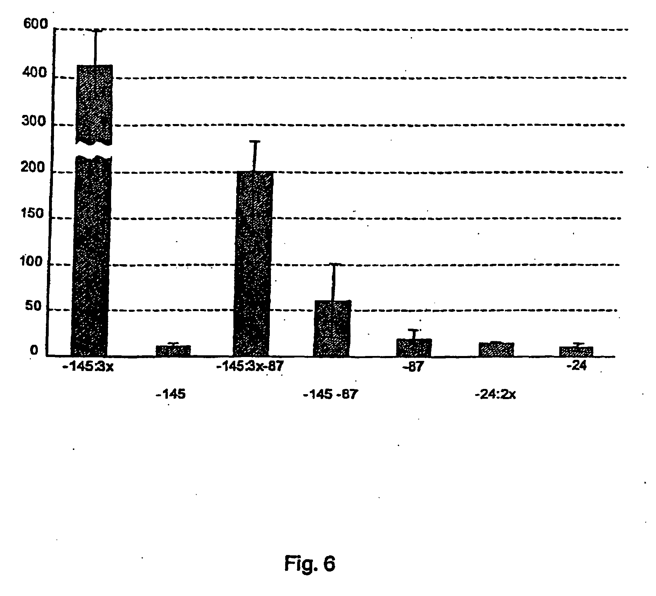

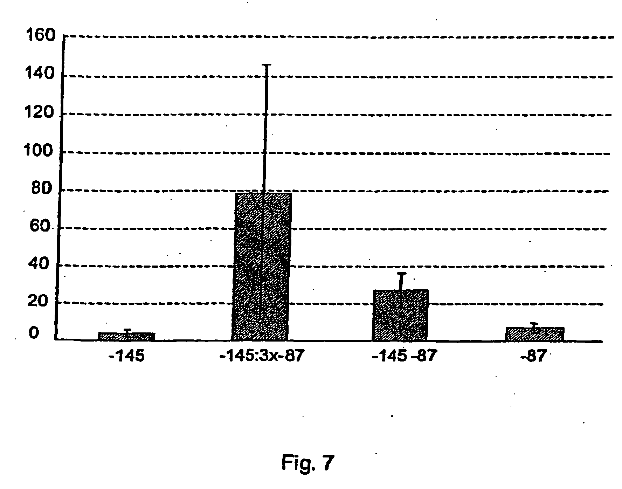

To test whether inhibition increases on placing various sites for binding to endogenous U1 snRNP in the same messenger, we include 3 binding sites at the -145 position (-145:3x), one at the -145 and another at the -87 (-145-87), and in the case of the reporter of renilla, two sites at the -24 position (RL-24:2x). In all cases the corresponding negative controls were constructed, as explained previously. HeLa cells were transfected in the manner described and the inhibition ratio was found (Fig. 6 and Fig. 7). Greatest inhibition was again obtained with the messenger of renilla: 450 times with RL-145:3x, 62 times with RL-145-87 and 14 times with RL-24:2x. Inhibition of the luciferase messenger was 27 times in LUC-145-87 and 78 times in LUC-145:3x-87. Such high inhibition ratios have not been encountered in the literature. Furthermore, this suggests that there is a cooperative effect in inhibition, so that various binding sites act much better than the sum of the inhibitions obtained for each one of them.

### 2. Modification of U1 snRNP so that it interacts with the 3' terminal exon of the reporter genes.

Once it had been ascertained that endogenous U1 snRNP is able to inhibit the expression of reporter genes, we tested whether modified U1 snRNP might be able to inhibit endogenous genes. Prior to this experiment, and for a more detailed analysis of the action of a modified U1 snRNP, its action on the 2 reporter genes (LUC and RL) was analyzed. For this, U1 snRNP was modified so that it interacted with the mutated reporter genes (LucM or RLM). As a negative control, a wild-type U1 snRNP was used at its 5' end (SEQ ID NO: 1), which was designated by the vague terms "wild-type non-endogenous" or U1-Mscl. This wild-type, non-endogenous U1 snRNP corresponds to the U1 snRNP modified at 4 positions of its 3' end that make it possible to evaluate its level of expression, differentially relative to endogenous U1 snRNP, using the technique for extension of an oligo "primer extension" as described in the literature (Primer extension. In "Molecular Cloning. A Laboratory Manual". Sambrook J., Fritsh E.F., Maniatis T. (Eds.) Cold Spring Harbor Laboratory Press. 1989, 2nd Ed., pp. 7.79-7.82). All the U1 snRNPs that were used had these 4 mutations which are not necessary for the functionality of the U1 snRNPs and do not constitute any limitation of the scope of the invention. The construct U1-Mut (SEQ ID NO: 2) was designed with 3 point mutations capable of hybridization with the sequences of LucM.

Experiments were carried out for cotransfection of U1-Mut (or if applicable U1-Mscl) simultaneously with each of the reporters LucM and RLM constructed, and the activity of luciferase and of renilla expressed in the presence of U1-Mscl was compared with the activities expressed in the presence of U1-Mut. The methodology of these experiments is similar to what was described previously.

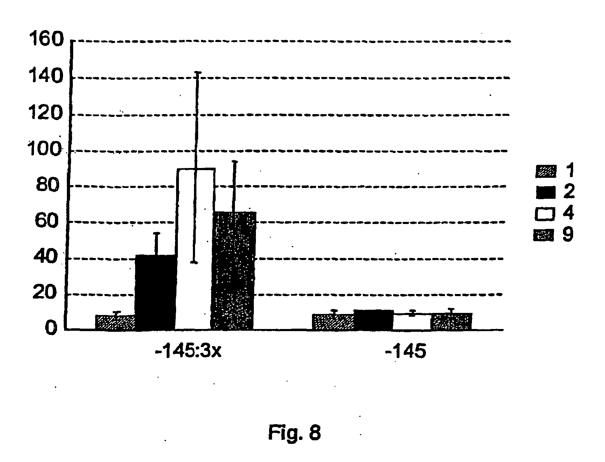

The results of cotransfection of U1-Mut (or U1-Mscl if applicable) with RL-145:3x and RL-145 are shown in Fig. 8, expressed as expression ratio. Also shown are the results obtained on cotransfecting with increasing doses (1, 2, 4 and 9) of exogenous U1 snRNP (U1-Mscl and U1-Mut).

With the minimum dose of U1 snRNP, ratios of inhibition of the activity of renilla of the order of 10 times were obtained. This is very similar to what had been obtained with the endogenous U1 snRNP in cases when this bound itself to the messenger at a single site. However, on increasing the transfected dose, there was an increase in the inhibition ratio in those messengers with various binding sites to U1 snRNP. Thus, for the messenger of renilla with three binding sites to U1 snRNP at the -145 position (RL-145:3x) inhibition ratios of 100 times were obtained. This is 4 times less than is obtained with the endogenous U1 snRNP. This may be due to the fact that the quantity of modified U1 snRNP expressed in these cells is less than that of the endogenous U1 snRNP. At dose 4, which has a greater effect, it was shown that the quantity of exogenous U1 snRNP expressed represents 17% relative to the endogenous U1 snRNP.

In this particular embodiment of the invention, the best results of inhibition obtained by antisense approximations described in the state of the art were multiplied by 5. On the other hand, in the majority of cases described in the literature, an inhibition of expression of up to 5 times means the loss of functionality of the gene. In very few cases have genes been described that continue to be functional when inhibitions of their expression of up to 10 times are obtained. No cases of genes that can be functional when their level of expression is 100 times less than the physiological level have been described.

### 3. Modification of U1 snRNP so that it interacts with the 3' terminal exon of endogenous genes of therapeutic interest.

Once we knew that the modified U1 snRNP can inhibit the expression of reporter genes, we tested whether it is able to inhibit the expression of endogenous genes. The endogenous genes chosen are those produced starting from the hepatitis B virus (HBV) both for their therapeutic interest and on the basis that they all use the same polyadenylation sequence. As the modified U1 snRNPs inhibit polyadenylation, if U1 snRNPs are designed so that they interact near the polyadenylation sequence of the RNAs of HBV, we should have to inhibit expression of all of the genes of HBV at the same time. Therefore U1 snRNP was modified so that it interacted with the terminal exon of the mRNAs transcribed by HBV. Inhibition of expression was greater when various modified snRNPs of U1 were used simultaneously, obtaining inhibitions of up to 5 times (see example 3).

The fact that the expression of endogenous genes can be inhibited using modified U1 snRNPs ensures that this technique is applicable both for studying gene function and for blocking the expression of toxic genes of therapeutic interest.

### Example 1: Introduction of the sequences recognizable by U1 snRNP in the 3' region of the reporter genes

The 5' end of the wild-type U1 snRNP interacts by base-pairing with the 5' sequences of the intron of the immature messenger RNAs (mRNAs) in the cell nucleus. This is the first step for elimination of the introns and maturation of the mRNAs. The sequences of U1 snRNA that can interact are the nucleotides from 1 to 10, complementary to the 5' sequences of the intron. If the 5' end of U1 snRNA is altered so that it is complementary to sequences of the 3' terminal exon of a messenger whose expression we are interested in inhibiting, the modified U1 snRNA will interact, also by base-pairing, with the complementary sequences of the 3' terminal exon of the target RNA. These interactions occur in the cell nucleus, very probably at the same time as transcription takes place.

In order to include the binding site to U1 snRNA, or the mutant site used as control, at the -145 position of the mRNA of renilla or of luciferase, the pRL-SV40 plasmid (Promega) or the pGL3-promoter (Promega, containing the sequences of luciferase under a promoter and SV40 polyadenylation sequences) was digested with Xba I. The kinase-treated and hybridized oligos SEQ ID NO: 3 and SEQ ID NO: 4 were included in this region in order to construct the binding site to U1 snRNA and SEQ ID NO: 5 and SEQ ID NO: 6 for constructing the binding site to mutated U1 snRNA. Thus, pRL-145UU1, pLUC-145UU1, pRL-145UU1M and pLUC-145UU1M, respectively, were constructed. The positive clones were verified by sequencing. Owing to the cloning characteristics, the messenger transcribed from these genes has sequences that are able to bind to U1 snRNA from nucleotide -145 to -157, both inclusive, taking as nucleotide 0 the first adenine of the first consensus sequence of polyadenylation. Within these sequences, the mutant site used as control is unable to interact at positions -147, -150 and -152 therefore the maximum consecutive sequence that hybridizes is of 5 nucleotides from position -153 to -157, both inclusive.

To include the binding site to U1 snRNA, or the mutant site used as control, at position -24 of the mRNA of renilla, the pRL-SpA plasmid (the construction of this plasmid is described later) was digested with Xba I where the oligos indicated in the preceding paragraph were introduced and the positive clones pRL-SpA-24UU1 and pRL-SpA-24UU1M were verified by sequencing. In this way, the messenger transcribed from these genes has sequences that are able to bind to the endogenous U1 snRNA from nucleotide - 24 to nucleotide -36, inclusively. Within these sequences, the mutant site used as control has non-complementary nucleotides in positions -26, -29 and -31.

In order to include the binding site to U1 snRNA, or the mutant site used as control, at the -87 position of the mRNA of renilla or of luciferase, the plasmid pRL-SV40 or the pGL3-promoter was digested with Xba I and BamH I, and the result of carrying out a crossed PCR using the oligos stated below was cloned between these sequences:
SEQ ID NO: 7 and SEQ ID NO: 8 for constructing pRL-87UU1 and pLUC-87UU1 respectively and SEQ ID NO: 9 and SEQ ID NO: 10 for constructing pRL-87UU1M and pLUC-87UU1M respectively.

To amplify the DNA of renilla the oligo:
SEQ ID NO: 11 was used

To amplify the 3' region of the messengers of renilla and luciferase we used the oligo:
SEQ ID NO: 12 was used

Crossed PCR was carried out in the following way: the plasmid pRL-SV40 was amplified with the oligo SEQ ID NO: 11 and with SEQ ID NO: 8 or SEQ ID NO: 10 to construct the plasmids that bind to U1 snRNA or the mutants, respectively. In a second PCR, the plasmid pRL-SV40 was used with SEQ ID NO: 7 or SEQ ID NO: 9 and SEQ ID NO: 12. The result of the two PCRs with sequences of binding to endogenous U1 snRNA or the mutated sequences were mixed independently and were amplified with SEQ ID NO: 11 and SEQ ID NO: 12. This third PCR produced fragments that were digested with Xba I and BamH I and were cloned at the same positions of pRL-SV40 or the pGL3-promoter. The positive clones were verified by sequencing and were designated pRL-87UU1 and pRL-87UU1M and pLUC-87UU1 and pLUC-87UU1M respectively. Accordingly, the messenger transcribed from these genes has sequences that are able to bind to endogenous U1 snRNA from nucleotide -87 to nucleotide -101 inclusively. Within these sequences, the mutant site used as control has non-complementary nucleotides at positions - 89, -92 and -94 therefore the largest consecutive sequence that hybridizes is of 7 nucleotides from position -95 to position -101 inclusively.

In order to include three binding sites to U1 snRNA, or the control sites, at position -145 of pRL-SV40, the procedure was followed as in the construction of pRL-145UU1 and pRL-145UU1M but instead of a single hybridized oligo, three were cloned in the same direction. The positive clones were verified by sequencing and were designated pRL-145:3xUU1 and pRL-145:3xUU1M. The messenger of renilla transcribed from the plasmid pRL-145:3xUU1 binds to the 5' end of the endogenous U1 snRNA at positions -145 to -157, -169 to -181 and -193 to -205, all inclusively. Within these sequences, the mutant site used as control has non-complementary nucleotides at positions -147, -150, -152, -171, -174, -176, -195, -198 and -200.

In order to include one binding site to U1 snRNA, or control sequence, at position -145 and a binding site or control at position -87 of pRL-SV40 and pGL3-promoter, we started from the plasmids pRL-87UU1 and pRL-87UU1M and pLUC-87UU1 and pLUC-87UU1M which were digested with Xba I and a hybridized oligo was cloned as in the construction of pRL-145UU1, pRL-145UU1M, pLUC-145UU1 and pLUC-145UU1M. The positive clones were verified by sequencing and were designated pRL-145-87UU1, pRL-145-87UU1M, pLUC-145-87UU1 and pLUC-145-87UU1M. In order to include 3 binding sites to U1 snRNA, or the control sites, at position -145 and a binding or control site at position -87 of pRL-SV40 and pGL3-promoter, we started from the plasmids pRL-87UU1 and pRL-87UU1M and pLUC-87UU1 and pLUC-87UU1M which were digested with Xba I and three hybridized oligos were cloned as in the construction of pRL-145:3xUU1 and pRL-195:3xUU1M. The positive clones were verified by sequencing and were designated pRL-145:3x-87UU1, pRL-145:3x-87UU1M, pLUC-145:3x-87UU1 and pLUC-145:3x-87UU1M.

In order to include 2 binding sites to U1 snRNA, or the control sites, at position -24 of pRL-SV40, we proceeded as in the construction of pRL-24UU1 and pRL-24UU1M but instead of a single hybridized oligo, two were cloned in the same direction. The positive clones were verified by sequencing and were designated pRL-24:2xUU1 and pRL-24:2xUU1M. The messenger of renilla transcribed from the plasmid pRL-24:2xUU1 binds to the 5' end of the endogenous U1 snRNA at positions -24 to -36 and -48 to -60, all inclusively. Within these sequences, the mutant site used as control has non-complementary nucleotides at positions -26, -29, -31, -50, -53 and -55.

### Cloning of the synthetic polyadenylation site in the reporter of renilla

In order to carry out this cloning we started from the pRL-SV40 gene (Promega). It was digested with BamH I and Xba I, eliminating the noncoding 3' end of the gene and the SV40 polyadenylation sequences. Into this region two oligos were introduced which, after hybridization together and treatment with kinase, have ends that are able to bind to sequences digested by BamH I and Xba I. The sequence of these oligos in the 5' to 3' direction is: SEQ ID NO: 13 and SEQ ID NO: 14.

### Transfection of HeLa, COS-1 and BHK cells with the gene constructions of luciferase and renilla

The calcium phosphate method was used for transfecting these cells. Confluent cells are diluted 5 times in M12 plates (with twelve wells 175 mm in diameter). On the next day their culture medium is changed and 4 hours later the precipitates of calcium phosphate together with the DNA are added. To form these precipitates, 1 microgram (in the case of the plasmids of renilla) or 8 micrograms (for the plasmids of luciferase) are mixed with 12.5 microlitres of a 2.5M solution of calcium chloride to a final volume of 125 microlitres. Then 125 microlitres of HBS 2x (280 mM NaCl, 50 mM Hepes and 1.5 mM Na₂HPO₄ at pH 7.12) are added. The mixture is incubated at room temperature for 20 minutes and 62.5 microlitres are added dropwise on each well in the plate. After 14-16 hours the plate is washed with serum-free medium and then medium with fresh serum is added. The expression of luciferase and of renilla luciferase is measured 48 hours after transfection using the "Dual luciferase reporter assay system" (Promega) in accordance with the manufacturer's recommendations. The data shown represent the mean of at least three experiments in each of which three separate wells, transfected with the same transfection mixture, were analyzed. This protocol was refined in several aspects: first, to ensure that the cell types used are transfected efficiently in this way; second, to analyze that, with the amounts of plasmid used, they are within a linear range of transfection; third, to determine that the method is reproducible. The time post-transfection when luciferase or renilla is measured coincides with the time of maximum accumulation of protein, though not with the time of the maximum degree of inhibition by U1 snRNA. Greater inhibitions were obtained at shorter times post-transfection.

### Transfection of increasing doses of plasmid to analyze whether the effect of U1 snRNA depends on the dose of mRNA

Normally 1 microgram of renilla plasmid is used in a transfection mixture (which corresponds to 0.25 micrograms of DNA per well in M12). To carry out this experiment, 1, 2, 4 and 8 micrograms of plasmid were used per transfection mixture. All of the doses are within the linear range of the method.

### Example 2: Construction of modified U1 snRNA

To construct the plasmid of U1 snRNA with mutations (pGem3z+:U1-Mut; SEQ ID NO: 2), in all cases we started from the plasmid pGem3z+:U1-Mscl (SEQ ID NO: 1) digested with BglI and BelI and the hybridized and kinase-treated oligos shown below were included between these sequences:
SEQ ID NO: 15
SEQ ID NO: 16

The positive clones were verified by sequencing.

### Cotransfection of HeLa cells with modified U1 snRNA

In the protocol for transfection of HeLa cells described previously (Example 1), the maximum quantity of DNA that can be transfected without altering the result of the transfection has been established. The result shows that it must not exceed 20 micrograms of DNA in the mixture described. On this basis the experiments were performed by cotransfecting increasing amounts of the plasmid of U1 snRNA while keeping the amount of plasmid of renilla and of luciferase constant. So that all the results would be comparable, always the same total plasmid quantity was transfected, using pGem3z+:U1-Mscl (SEQ ID NO: 1). The quantities of modified U1 snRNA plasmid used were 1, 2, 4 and 9 micrograms. To ensure an adequate efficiency of cotransfection, a plasmid was constructed that contained the sequences of renilla and the modified U1 snRNA or the control. For this, pRL-145UU1M, pRL-145:3xUU1M, pRL-145-87UU1M, pRL-87UU1M and pRL-24:2xUU1M were digested with BamH I and pGem3z+:U1-Mscl (SEQ ID NO: 1) or pGem3z+:U1-Mut (SEQ ID NO: 2) digested with BamH I was introduced. The different orientation of the sequences of U1 snRNA relative to the messenger of renilla did not produce differences in the quantity of U1 snRNA expressed in the cell. Transfection of these plasmids in those with co-expression of the gene of renilla and that of U1 snRNA produced similar results to the cotransfection of the plasmids of renilla and U1 snRNA separately.

### Example 3: Inhibition of mRNAs using modified U1 snRNPs for therapeutic purposes using the hepatitis B virus as a model

The hepatitis B virus has a genome in the form of circular DNA. Transcription of the viral genes starts from separate sites of the genome but they all have the same polyadenylation sequences, so that action directed against this region should lead to inhibition of expression of all the viral mRNAs. Therefore we constructed four modified U1s of nucleotides 1 to 14 of its 5' end. We replaced these sequences with sequences that hybridize to 36, 72, 89 and 127 nucleotides upstream of the polyadenylation site. We thus obtain UHB35 (SEQ ID NO: 17), UHB72 (SEQ ID NO: 18), UHB89 (SEQ ID NO: 19) and UHB127 (SEQ ID NO: 20). It was found that these sequences are identical in the majority of strains of human hepatitis B. A cryptic polyadenylation site has also been described in this virus. The polyadenylation site of HBV that is generally used is at position 1918 of pHBV, its sequence is TATAAA, and not exactly the consensus sequence AATAAA. If it does not have the consensus sequence and if there are difficulties in using this sequence, another site is used, of sequence CATAAA, located at position 1790 of the pHBV plasmid. As this site is not usually used and it does not have the consensus sequence, it is called cryptic, i.e. it should not be used but the machinery of polyadenylation sometimes uses it. The last of the modified U1s at 127 nucleotides of the usual polyadenylation site will be directed against this sequence.

All the plasmids are in the vector pGem 3 and they originated from a vector pGem3z+:U1-WY that incorporates the sequence of endogenous human U1 snRNA (NCBI V00591). The sequences were introduced in this plasmid at the BamH I site and with the 5' end of the gene next to the T7 site. The plasmids shown are DNA, therefore the promoter sequences, the terminator sequences and what will actually be transcribed are included there. The modifications are located in the first 15 nucleotides of the fragment that is transcribed.
SEQ ID NO: 17 pGem3z+U1 HB35. Interacts with position -35 of hepatitis B (regarding the polyadenylation site as position 0).
SEQ ID NO: 18 pGem3z+U1 HB72. Interacts with position -72 of hepatitis B (regarding the polyadenylation site as position 0).
SEQ ID NO: 19 pGem3z+U1 HB89. Interacts with position -89 of hepatitis B (regarding the polyadenylation site as position 0).
SEQ ID NO: 20 pGem3z+U1 HB127. Interacts with position -127 of hepatitis B (regarding the polyadenylation site as position 0).

For constructing the plasmids of U1 with mutations, in all cases we started from the plasmid pGem3z+:U1-Mscl (SEQ ID NO: 1) digested with Bgl II and Bcl I and between these sequences were included the hybridized and kinase-treated oligos indicated below. In all cases the positive clones were verified by sequencing.
a) For constructing pGem3z+U1 HB35 the oligos used were:
   HB-355:5' (SEQ ID NO: 21)
   HB-353:5' (SEQ ID NO: 22)
   The clone of U1 that was obtained is able to hybridize with the 3' end of the messengers of HBV from position -36 to position -51 inclusively.
b) For constructing pGem3z+U1 HB70 the oligos used were:
   HB-705:5' (SEQ ID NO: 23)
   HB-703:5' (SEQ ID NO: 24)
   The clone of U1 that was obtained is able to hybridize with the 3' end of the messengers of HBV from position -72 to position -88 inclusively.
c) For constructing pGem3z+U1 HB89 the oligos used were:
   HB-895:5' (SEQ ID NO: 25)
   HB-893:5' (SEQ ID NO: 26)
   The clone of U1 that was obtained is able to hybridize with the 3' end of the messengers of HBV from position -89 to position -104 inclusively.
d) For constructing pGem3z+U1 HB125 the oligos used were:
   HB-1255:5' (SEQ ID NO: 27)
   HB-1253:5' (SEQ ID NO: 28)
   The clone of U1 that was obtained is able to hybridize with the 3' end of the messengers of HBV from position -127 to position -140 inclusively.

For testing this system we have at our disposal a cell line designated 2215, which contains the integrated viral genome and has normally been used for testing the effect of drugs on expression of this virus. The cell line 2215 is a derivative of the HepG2 line and contains the HBV genome, subtype AYW, shown in the sequence SEQ ID NO: 29.

Information on this cell line is given in: Hepatitis B virus cell culture assays for antiviral activity. Methods in Molecular Medicine, Vol. 24: Antiviral methods and protocols. Edited by D. Kinchington and R.F. Schinazi. Humana Press Inc., Totowa, NJ. Using this system, we tested the effect of one or more U1s on expression of the viral genes. The cell line is described in: "Hepatitis B virus cell culture assays for antiviral activity". K. Schmidt and B. Korba. Methods in Molecular Medicine, Vol. 24. Antiviral methods and protocols, pp. 51-67.

In order to analyze the effect of the modified U1 snRNPs on these cells, we transfected the latter using the calcium phosphate method. The cells are cultivated on M6 plates (with six wells, 35 mm in diameter), on the next day their culture medium is changed and 4 hours later the precipitates of calcium phosphate together with the DNA are added. To form these precipitates, 4 micrograms of each of the 4 plasmids that express the modified U1 snRNPs or 16 micrograms of the control plasmid that expresses the wild-type U1 snRNP are mixed with 12.5 microlitres of a 2.5M solution of calcium chloride to a final volume of 125 microlitres. If from 1 to 3 plasmids are being transfected, 4 micrograms of each of them are used and the remainder, up to 16 micrograms, is made up with an irrelevant plasmid. We thus ensure that the final concentration of DNA is the same in all the transfections. Furthermore, in all cases, 2 micrograms of the pMac plasmid (see later) are added, permitting selection of the transfected cells. 125 microlitres of HBS 2x (280 mM NaCl, 50 mM Hepes and 1.5 mM Na₂HPO₄ at pH 7.12) are added to the mixtures of DNA with CaCl. The mixture is incubated at room temperature for 20 minutes, and 62.5 microlitres are added dropwise to each well in the plate. After a further 14-16 hours, the plate is washed with serum-free medium, and medium with fresh serum is added.

48 hours after transfection, we analyze whether inhibition of expression of viral proteins has occurred. The efficiency of transfection of the 2215 cells by the calcium phosphate method is only 10%. Other methods of transfection that were tested proved less effective. Therefore it is necessary to select the transfected cells, and for this purpose we used the pMac plasmid (Miltenyi) that expresses a membrane protein that is recognizable by an antibody coupled to a magnetic ball that is selected using a magnetized column. We had previously ensured that the efficiency of cotransfection is above 95%. The selected cells are lysed and the quantities of surface antigen (AgS) and antigen E (AgE), two proteins of the hepatitis B virus, are measured using a commercial ELISA technique (Roche). The result is given in the following table, which only shows the combinations of modified U1 snRNAs with which the greatest inhibitions were obtained.

| | AgS | AgE |
|---|---|---|
| Wild-type U1 snRNP | 1357 | 1256 |
| UHB35 + UHB89 | 1295 | 631 |
| UHBs (35, 72, 89 and 127) | 667 | 298 |

### DESCRIPTION OF THE FIGURES

- Fig. 1.: Maturation of the mRNA in the nucleus. I - DNA; II - RNA; III - Polymerase II; IV - pre-mRNA; V - mRNA; VI - Nucleus; VII - Cytoplasm; 1 - Transcription; 2 - Addition of the cap to the 5' end; 3 - Maturation by cutting and splicing; 4 - Polyadenylation; 5 - Transport; 6 - Translation.
- Fig. 2A.: Sketch of the structure of U1 snRNA. 1 - Region of binding to 5; 2 - Loop A; 3 - Loop B; 4 - Loop C; 5 - Region of binding to Sm; 6 - Loop D.
- Fig. 2B.: Structure of U1 snRNP. 1 - 70K; 2 - U1A; 3 - U1C; 4 - Sm
- Fig. 3.: General diagram of the 3' end of the messenger RNA. a - Immature messenger RNA; b - mature messenger RNA; 1 - 3' terminal exon; 2 - Polyadenylation sequence; 3 - First consensus polyadenylation sequence; 4 - Second consensus polyadenylation sequence; 5 - Noncoding 3' end; A - 3' end of the terminal intron; B - Translation stop codon; C - Position 0; D - Site of rupture and polyadenylation; I - Upstream direction; II - Downstream direction.
- Fig. 4A.: Interaction of endogenous U1 (U1 snRNP WT) with mRNA of luciferase (LUC).
- Fig. 4B.: Interaction of endogenous U1 with the mRNA of mutated luciferase (LUCM) used as negative control. Three point mutations (shown bold and underlined) have been introduced into the sequence for binding to U1, so that binding is not functional.
- Fig. 5.: Ratio of inhibition of gene expression of renilla (RL) and luciferase (LUC) with endogenous U1 in HeLa cells. The binding sites tested are shown on the abscissa. The ordinate shows the inhibition ratio: activity of RLM/RL or LucM/LUC respectively.
- Fig. 6.: Ratio of inhibition of expression of renilla (RLM/RL) with various binding sites to U1, shown on the abscissa.
- Fig. 7.: Ratio of inhibition of expression of luciferase (LucM/LUC) with various binding sites to U1, shown on the abscissa.
- Fig. 8.: Ratio of inhibition of the expression of renilla (U1-Mscl / U1-Mut) with increasing doses (1, 2, 4 and 9 doses respectively) of exogenous U1 snRNP, both for RL-145:3x and for RL-145 (on the abscissa).

## Claims

1. A method of reversible inhibition of gene expression, **characterized in that** the RNA of at least one ribonucleoprotein U1 snRNP with at least one modification at the 5' end of its nucleotide sequence, is hybridized in at least one region of the 3' terminal exon of a target mRNA of the gene to be inactivated.

2. The method of reversible inhibition of gene expression according to claim 1, **characterized in that** the nucleotides of the 5' end of the RNA of the ribonucleoprotein U1 snRNP involved in the hybridization with the target mRNA of the gene to be inactivated are some of the first 15 of said 5' end.

3. The method of reversible inhibition of gene expression according to claims 1 and 2, **characterized in that** the regions of hybridization of the 5' end of the RNA of U1 snRNP at the 3' terminal exon of the mRNA of the gene to be inactivated are upstream or downstream always with respect to the first consensus polyadenylation sequence of the mRNA.

4. The method of reversible inhibition of gene expression according to claims 1 to 3, **characterized in that** the U1 snRNPs that bind together in different regions of hybridization are between 1 and 4.

5. A modified U1 snRNP ribonucleoprotein for reversibly inhibiting gene expression, **characterized in that** said ribonucleoprotein can hybridize with a sequence included within the 3' terminal exon of the target mRNA of the gene to be inactivated.

6. The modified U1 snRNP ribonucleoprotein for reversibly inhibiting gene expression according to claim 5, **characterized in that** said ribonucleoprotein can hybridize with a sequence larger than 7 nucleotides, preferably between 7 and 15, contained within the 3' terminal exon of the target mRNA of the gene to be inactivated.

7. The modified U1 snRNP ribonucleoprotein for reversibly inhibiting gene expression according to claims 5 and 6, **characterized in that** the modification of said ribonucleoprotein comprises at least 1 of the first 15 nucleotides of the 5' end.

8. An expression vector containing DNA sequences that code for modified U1 snRNPs according to claims 5 to 7.

9. The expression vector according to claim 8, **characterized in that** it is a plasmid.

10. The expression vector according to claim 9, **characterized in that** it contains SEQ ID NO: 1.

11. The expression vector according to claim 9, **characterized in that** it contains SEQ ID NO: 2.

12. The expression vector according to claim 9, **characterized in that** it contains SEQ ID NO: 17.

13. The expression vector according to claim 9, **characterized in that** it contains SEQ ID NO: 18.

14. The expression vector according to claim 9, **characterized in that** it contains SEQ ID NO: 19.

15. The expression vector according to claim 9, **characterized in that** it contains SEQ ID NO: 20.

16. A cell line, **characterized in that** it has been transformed with any of the vectors of claims 8 to 15.

17. The cell line according to claim 16, **characterized in that** it comprises HeLa cells.

18. The cell line according to claim 16, **characterized in that** it comprises COS-1 cells.

19. The cell line according to claim 16, **characterized in that** it comprises BHK cells.

20. The cell line according to claim 16, **characterized in that** it comprises 2215 cells.

21. Any application of the method of claims 1 to 4, of the ribonucleoproteins of claims 5 to 7 and/or of the vectors of claims 8 to 9 in the functional investigation of genes of unknown function.

22. Any application of the method of claims 1 to 4, of the ribonucleoproteins of claims 5 to 7 and/or of the vectors of claims 8 to 15 in the functional investigation of genes of the hepatitis B virus.

23. Use according to claims 21 or 22, **characterized in that** it employs tissue culture systems.

24. Use of the ribonucleoproteins of claims 5 to 7 and/or of the vectors of claims 8, 9, 10 and 12 to 15, together with pharmaceutically acceptable excipients, in the manufacture of compositions that can be used in somatic gene therapy.

25. Use of the method of claims 1 to 4 in treatments of somatic gene therapy.

26. Use of the method of claims 1 to 4 in the treatment of infections that can be attributed to the hepatitis B virus.

27. Any application of the ribonucleoproteins of claims 5 to 7 and/or of the vectors of claims 8, 9, 10 and 12 to 15, together with pharmaceutically acceptable excipients, in the manufacture of compositions that can be used in the treatment of infections that can be attributed to the hepatitis B virus.


SEQUENCE LISTING

<110> INSTITUTO CIENTÍFICO Y TECNOLÓGICO DE NAVARRA, S.A.
<120> "METHOD OF REVERSIBLE INHIBITION OF GENE EXPRESSION
BY MEANS OF MODIFIED RIBONUCLEOPROTEINS"
<130> PCT-109
<150> ES 200100765
<151> 2001-03-30
<160> 29
<170> PatentIn version 3.1
<210> 1
<211> 607
<212> DNA
<213> artificial sequence
<220>
<223> Control plasmid pGem3z+:U1-Mscl (non-endogenous
wild-type U1 snRNA). Incorporates the sequence for
the transcription of a modified endogenous U1 snRNA
at 4 positions of its 3' end
<220>
<221> promoter
<222> (9)..(400)
<223>
<220>
<221> characteristic
<222> (401)..(469)
<223> Sequence for transcription of U1 snRNA-Mscl
<220>
<221> termination
<222> (570)..(602)
<223> Transcription terminating sequence
<220>
<221> modified base
<222> (498)..(499)

<223>  Modified bases relative to endogenous U1 snRNA

<220>

<221>  modified base

<222>  (509)..(510)

<223>  Modified bases relative to endogenous U1 snRNA

<400>  1
ggatccggta aggaccagct tctttgggag agaacagacg caggggcggg agggaaaaag   60
ggagaggcag acgtcacttc cccttggcgg ctctggcagc agattggtcg gttgagtggc  120
agaaaggcag acggggactg ggcaaggcac tgtcggtgac atcacggaca gggcgacttc  180
tatgtagatg aggcagcgca gaggctgctg cttcgccact tgctgcttca ccacgaagga  240
gttcccgtgc cctgggagcg ggttcaggac cgctgatcgg aagtgagaat cccagctgtg  300
tgtcagggct ggaaagggct cgggagtgcg cggggcaagt gaccgtgtgt gtaaagagtg  360
aggcgtatga ggctgtgtcg gggcagaggc ccaagatctc atacttacct ggcaggggag  420
ataccatgat cacgaaggtg gttttcccag ggcgaggctt atccattgca ctccggatgt  480
gctgacccct gcgatttggc caaatgtgcc aaactcgact gcataatttg tggtagtggg  540
ggactgcgtt cgcgctttcc cctgactttc tggagtttca aaagtagact gtacgctaac  600
cggatcc                                                             607

<210>  2

<211>  607

<212>  DNA

<213>  artificial sequence

<220>

<223> Plasmid inhibiting pGem3z+:U1-Mut reporter genes.
Derived from the pGem3z+:U1-Mscl plasmid by modification
of 3 bases at the 5' end of U1 snRNA

<220>

<221>  modified base

<222>  (404)..(404)

<223>

<220>

<221>  modified base

<222>  (407)..(407)

<223>

<220>

<221>  modified base

<222>  (409)..(409)

<223>

<400>  2

ggatccggta aggaccagct tctttgggag agaacagacg caggggcggg agggaaaaag   60

ggagaggcag acgtcacttc cccttggcgg ctctggcagc agattggtcg gttgagtggc  120

agaaaggcag acggggactg ggcaaggcac tgtcggtgac atcacggaca gggcgacttc  180

tatgtagatg aggcagcgca gaggctgctg cttcgccact tgctgcttca ccacgaagga  240

gttcccgtgc cctgggagcg ggttcaggac cgctgatcgg aagtgagaat cccagctgtg  300

tgtcagggct ggaaagggct cgggagtgcg cggggcaagt gaccgtgtgt gtaaagagtg  360

aggcgtatga ggctgtgtcg gggcagaggc ccaagatctc atagttccat ggcaggggag  420

ataccatgat cacgaaggtg gttttcccag ggcgaggctt atccattgca ctccggatgt  480

gctgacccct gcgatttggc caaatgtgcc aaactcgact gcataatttg tggtagtggg  540

ggactgcgtt cgcgctttcc cctgactttc tggagtttca aaagtagact gtacgctaac  600

cggatcc                                                            607

<210>  3

<211>  24

<212>  DNA

<213>  artificial sequence

<220>

<223>  Oligonucleotide

<400>  3

ctagatgcca ggtaagtaaa gtgt                                          24

<210>  4

<211>  24

<212>  DNA

<213>  artificial sequence

<220>

<223>  Oligonucleotide

<400>  4
ctagacactt tacttacctg gcat                                              24

<210>  5
<211>  24
<212>  DNA
<213>  artificial sequence
<220>
<223>  Oligonucleotide

<400>  5
ctagatgcca tggaactaaa gtgt                                              24

<210>  6
<211>  24
<212>  DNA
<213>  artificial sequence
<220>
<223>  Oligonucleotide

<400>  6
ctagacactt tagttccatg gcat                                              24

<210>  7
<211>  40
<212>  DNA
<213>  artificial sequence
<220>
<223>  Oligonucleotide

<400> 7

acctgccagg taagtaaagt gacaactaga atgcagtgaa 40

<210> 8
<211> 40
<212> DNA
<213> Artificial Sequence
<220>
<223> Oligonucleotide

<400> 8
ttgtcacttt acttacctgg caggtttgtc caaactcatc 40

<210> 9
<211> 40
<212> DNA
<213> artificial sequence
<220>
<223> Oligonucleotide

<400> 9
acctgccatg gaactaaagt gacaactaga atgcagtgaa 40

<210> 10
<211> 40
<212> DNA
<213> artificial sequence
<220>
<223> Oligonucleotide

<400>  10
ttgtcacttt agttccatgg caggtttgtc caaactcatc                    40

<210>  11
<211>  20
<212>  DNA
<213>  artificial sequence
<220>
<223>  Oligonucleotide

<400>  11
gatgcacctg atgaaatggg                                          20

<210>  12
<211>  20
<212>  DNA
<213>  artificial sequence
<220>
<223>  Oligonucleotide

<400>  12
cgcacatttc cccgaaaagt                                          20

<210>  13
<211>  74
<212>  DNA
<213>  artificial sequence
<220>
<223>  Oligonucleotide

<400> 13
ctagagatcc cgaattcaat aaagagctct tattttcatt ctcgaggtgt ggttggtttt 60
tttgtgtggg ggcg 74

<210> 14
<211> 74
<212> DNA
<213> artificial sequence
<220>
<223> Oligonucleotide

<400> 14
gatccgcccc cacacaaaaa aaccaaccac acctcgagaa tgaaaataag agctctttat 60
tgaattcggg atct 74

<210> 15
<211> 33
<212> DNA
<213> artificial sequence
<220>
<223> Oligonucleotide U1mut5

<400> 15
gatctcatag ttccatggca ggggagatac cat 33

<210> 16
<211> 33
<212> DNA
<213> artificial sequence
<220>
<223> Oligonucleotide U1mut3

<400>  16
gatcatggta tctcccctgc catggaacta tga                                33

<210>  17
<211>  607
<212>  DNA
<213>  artificial sequence
<220>
<223> pGem3z+U1 HB35 inhibition plasmid. Derived from the
plasmid pGem3z+:U1-Mscl by modification in the first 15
bases of the 5' end of U1 snRNA for hybridizing with the
3' end of the HBV messengers from position -36 to -51
(regarding the polyadenylation site as position 0)
<220>
<221>  modified base
<222>  (402)..(415)
<223>  Modified bases relative to pGem3z+:U1-Mscl

<400>  17
ggatccggta aggaccagct tctttgggag agaacagacg caggggcggg agggaaaaag   60
ggagaggcag acgtcacttc cccttggcgg ctctggcagc agattggtcg gttgagtggc  120
agaaaggcag acggggactg ggcaaggcac tgtcggtgac atcacggaca gggcgacttc  180
tatgtagatg aggcagcgca gaggctgctg cttcgccact tgctgcttca ccacgaagga  240
gttcccgtgc cctgggagcg ggttcaggac cgctgatcgg aagtgagaat cccagctgtg  300
tgtcagggct ggaaagggct cgggagtgcg cggggcaagt gaccgtgtgt gtaaagagtg  360
aggcgtatga ggctgtgtcg gggcagaggc ccaagatctc acagcttgga ggcttgggag  420
ataccatgat cacgaaggtg gttttcccag ggcgaggctt atccattgca ctccggatgt  480
gctgacccct gcgatttggc caaatgtgcc aaactcgact gcataatttg tggtagtggg  540
ggactgcgtt cgcgctttcc cctgactttc tggagtttca aaagtagact gtacgctaac  600
cggatcc                                                             607

<210>  18
<211>  607
<212>  DNA

<213>  artificial sequence
<220>
<223> pGem3z+U1 HB72 inhibition plasmid. Derived from the
plasmid pGem3z+:U1-Mscl by modification in the first 15
bases of the 5' end of U1 snRNA for hybridizing with the
3' end of the HBV messengers from position -72 to -88
(regarding the polyadenylation site as position 0)
<220>
<221>  modified base
<222>  (402)..(415)
<223>  Modified bases relative to pGem3z+:U1-Mscl

<400>  18
ggatccggta aggaccagct tctttgggag agaacagacg caggggcggg agggaaaaag   60
ggagaggcag acgtcacttc cccttggcgg ctctggcagc agattggtcg gttgagtggc  120
agaaaggcag acggggactg ggcaaggcac tgtcggtgac atcacggaca gggcgacttc  180
tatgtagatg aggcagcgca gaggctgctg cttcgccact tgctgcttca ccacgaagga  240
gttcccgtgc cctgggagcg ggttcaggac cgctgatcgg aagtgagaat cccagctgtg  300
tgtcagggct ggaaagggct cgggagtgcg cggggcaagt gaccgtgtgt gtaaagagtg  360
aggcgtatga ggctgtgtcg gggcagaggc ccaagatctc agatgattag gcagagggag  420
ataccatgat cacgaaggtg gttttcccag ggcgaggctt atccattgca ctccggatgt  480
gctgacccct gcgatttggc caaatgtgcc aaactcgact gcataatttg tggtagtggg  540
ggactgcgtt cgcgctttcc cctgactttc tggagtttca aaagtagact gtacgctaac  600
cggatcc                                                            607

<210>  19
<211>  607
<212>  DNA
<213>  artificial sequence
<220>
<223> pGem3z+U1 HB89 inhibition plasmid. Derived from the
plasmid pGem3z+:U1-Mscl by modification in the first 15
bases of the 5' end of U1 snRNA for hybridizing with the 3'
end of the HBV messengers from position -89 to -104
(regarding the polyadenylation site as position 0)

<220>
<221> modified base
<222> (402)..(415)
<223> Modified bases relative to pGem3z+:U1-Mscl

<400> 19
ggatccggta aggaccagct tctttgggag agaacagacg caggggcggg agggaaaaag 60
ggagaggcag acgtcacttc cccttggcgg ctctggcagc agattggtcg gttgagtggc 120
agaaaggcag acggggactg ggcaaggcac tgtcggtgac atcacggaca gggcgacttc 180
tatgtagatg aggcagcgca gaggctgctg cttcgccact tgctgcttca ccacgaagga 240
gttcccgtgc cctgggagcg ggttcaggac cgctgatcgg aagtgagaat cccagctgtg 300
tgtcagggct ggaaagggct cgggagtgcg cggggcaagt gaccgtgtgt gtaaagagtg 360
aggcgtatga ggctgtgtcg gggcagaggc ccaagatctc atgaaaaagt tgcatgggag 420
ataccatgat cacgaaggtg gttttcccag ggcgaggctt atccattgca ctccggatgt 480
gctgacccct gcgatttggc caaatgtgcc aaactcgact gcataatttg tggtagtggg 540
ggactgcgtt cgcgctttcc cctgactttc tggagtttca aaagtagact gtacgctaac 600
cggatcc 607

<210> 20
<211> 607
<212> DNA
<213> artificial sequence
<220>
<223> pGem3z+U1 HB127 inhibition plasmid. Derived from the
plasmid pGem3z+:U1-Mscl by modification in the first 15
bases of the 5' end of U1 snRNA for hybridizing with the
3' end of the HBV messengers from position -127 to -140
(regarding the polyadenylation site as position 0)
<220>
<221> modified base
<222> (402)..(415)
<223> Modified bases relative to pGem3z+:U1-Mscl

<400>  20
   ggatccggta aggaccagct tctttgggag agaacagacg caggggcggg agggaaaaag 60
   ggagaggcag acgtcacttc cccttggcgg ctctggcagc agattggtcg gttgagtggc 120
   agaaaggcag acggggactg ggcaaggcac tgtcggtgac atcacggaca gggcgacttc  180
   tatgtagatg aggcagcgca gaggctgctg cttcgccact tgctgcttca ccacgaagga  240
   gttcccgtgc cctgggagcg ggttcaggac cgctgatcgg aagtgagaat cccagctgtg  300
   tgtcagggct ggaaagggct cgggagtgcg cggggcaagt gaccgtgtgt gtaaagagtg  360
   aggcgtatga ggctgtgtcg gggcagaggc ccaagatctc atgcctacag cctccgggag  420
   ataccatgat cacgaaggtg gttttcccag ggcgaggctt atccattgca ctccggatgt  480
   gctgacccct gcgatttggc caaatgtgcc aaactcgact gcataatttg tggtagtggg  540
   ggactgcgtt cgcgctttcc cctgactttc tggagtttca aaagtagact gtacgctaac  600
   cggatcc                                                            607

<210>  21

<211>  33

<212>  DNA

<213>  artificial sequence

<220>

<223> Oligonucleotide HB-355 (Used for the construction of
pGem3z+U1 HB35 from pGem3z+:U1-Mut)

<400>  21

gatcatggta tctcccaagc ctccaagctg tga                    33

<210>  22

<211>  33

<212>  DNA

<213>  artificial sequence

<220>

<223>  Oligonucleotide HB-353 (Used for the construction
of pGem3z+U1 HB35 from pGem3z+:U1-Mut)

<400>  22

gatctcacag cttggaggct tgggagatac cat                    33

<210> 23
<211> 33
<212> DNA
<213> artificial sequence
<220>
<223> Oligonucleotide HB-705 (Used for the construction
of pGem3z+U1 HB70 from pGem3z+:U1-Mut)

<400> 23
gatcatggta tctccctctg cctaatcatc tga 33

<210> 24
<211> 33
<212> DNA
<213> artificial sequence
<220>
<223> Oligonucleotide HB-703 (Used for the construction
of pGem3z+U1 HB70 from pGem3z+:U1-Mut)

<400> 24
gatctcagat gattaggcag agggagatac cat 33

<210> 25
<211> 33
<212> DNA
<213> artificial sequence
<220>
<223> Oligonucleotide HB-895 (Used for the construction
of pGem3z+U1 HB89 from pGem3z+:U1-Mut)
<400> 25
gatcatggta tctcccatgc aactttttca tga 33

<210>  26
<211>  33
<212>  DNA
<213>  artificial sequence
<220>
<223>  Oligonucleotide HB-893 (Used for the construction
of pGem3z+U1 HB89 from pGem3z+:U1-Mut)

<400>  26
gatctcatga aaaagttgca tgggagatac cat                                33

<210>  27
<211>  33
<212>  DNA
<213>  artificial sequence
<220>
<223>  Oligonucleotide HB-1255 (Used for the construction
of pGem3z+U1 HB125 from pGem3z+:U1-Mut)

<400>  27
gatcatggta tctcccggag gctgtaggca tga                                33

<210>  28
<211>  33
<212>  DNA
<213>  artificial sequence
<220>

<223> Oligonucleotide HB-1253 (Used for the construction
of pGem3z+U1 HB125 from pGem3z+:U1-Mut)

<400> 28
gatctcatgc ctacagcctc cgggagatac cat 33

<210> 29
<211> 3221
<212> DNA
<213> artificial sequence
<220>
<223> Sequence of HBV subtype AYW present in the 2115
cells
<220>
<221> characteristic
<222> (1918)..(1923)
<223> First polyadenylation sequence
<220>
<221> characteristic
<222> (1917)..()
<223> Reference position -1

<400> 29
aattccactg ccttgcacca agctctgcag gatcccagag tcaggggtct gtatcttcct 60
gctggtggct ccagttcagg aacagtaaac cctgctccga atattgcctc tcacatctcg 120
tcaatctccg cgaggactgg ggaccctgtg acgatcatgg agaacatcac atcaggattc 180
ctaggacccc tgctcgtgtt acaggcgggg tttttcttgt tgacaagaat cctcacaata 240
ccgcagagtc tagactcgtg gtggacttct ctcaattttc tagggggatc acccgtgtgt 300
cttggccaaa attcgcagtc cccaacctcc aatcactcac caacctcctg tcctccaatt 360
tgtcctggtt atcgctggat gtgtctgcgg cgttttatca tattcctctt catcctgctg 420
ctatgcctca tcttcttatt ggttcttctg gattatcaag gtatgttgcc cgtttgtcct 480
ctaattccag gatcaacaac aaccagtacg ggaccatgca aaacctgcac gactcctgct 540
caaggcaact ctaagtttcc ctcatgttgc tgtacaaaac ctacggatgg aaattgcacc 600
tgtattccca tcccatcgtc ctgggctttc gcaaaatacc tatgggagtg ggcctcagtc 660

cgtttctctt ggctcagttt actagtgcca tttgttcagt ggttcgtagg gctttccccc 720
actgtttggc tttcagctat atggatgatg tggtattggg ggccaagtct gtacagcatc 780
gtgagtccct ttataccgct gttaccaatt ttcttttgtc tctgggtata catttaaacc 840
ctaacaaaac aaaaagatgg ggttattccc taaacttcat gggctacata attggaagtt 900
ggggaacttt gccacaggat catattgtac aaaagatcaa acactgtttt agaaaacttc 960
ctgttaacag gcctattgat tggaaagtat gtcaaagaat tgtgggtctt ttgggctttg 1020
ctgctccatt tacacaatgt ggatatcctg ccttaatgcc tttgtatgca tgtatacaag 1080
ctaaacaggc tttcactttc tcgccaactt acaaggcctt tctaagtaaa cagtacatga 1140
acctttaccc cgttgctcgg caacggcctg gtctgtgcca agtgtttgct gacgcaaccc 1200
ccactggctg gggcttggcc ataggccatc agcgcatgcg tggaaccttt gtggctcctc 1260
tgccgatcca tactgcggaa ctcctagccg cttgttttgc tcgcagccgg tctggagcaa 1320
agctcatcgg aactgacaat tctgtcgtcc tctcgcggaa atatacatcg tttccatggc 1380
tgctaggctg tactgccaac tggatccttc gcgggacgtc ctttgtttac gtcccgtcgg 1440
cgctgaatcc cgcggacgac ccctctcggg gccgcttggg actctctcgt ccccttctcc 1500
gtctgccgtt ccagccgacc acggggcgca cctctcttta cgcggtctcc ccgtctgtgc 1560
cttctcatct gccggtccgt gtgcacttcg cttcacctct gcacgttgca tggagaccac 1620
cgtgaacgcc catcagatcc tgcccaaggt cttacataag aggactcttg gactcccagc 1680
aatgtcaacg accgaccttg aggcctactt caaagactgt gtgtttaagg actgggagga 1740
gctgggggag gagattaggt taaaggtctt tgtattagga ggctgtaggc acaaattggt 1800
ctgcgcacca gcaccatgca actttttcac ctctgcctaa tcatctcttg tacatgtccc 1860
actgttcaag cctccaagct gtgccttggg tggctttggg gcatggacat tgacccttat 1920
aaagaatttg gagctactgt ggagttactc tcgtttttgc cttctgactt ctttccttcc 1980
gtcagagatc tcctagacac cgcctcagct ctgtatcgag aagccttaga gtctcctgag 2040
cattgctcac ctcaccatac tgcactcagg caagccattc tctgctgggg ggaattgatg 2100
actctagcta cctgggtggg taataatttg gaagatccag catctaggga tcttgtagta 2160
aattatgtta atactaacgt gggtttaaag atcaggcaac tattgtggtt tcatatatct 2220
tgccttactt ttggaagaga gactgtactt gaatatttgg tctctttcgg agtgtggatt 2280
cgcactcctc cagcctatag accaccaaat gcccctatct tatcaacact tccggaaact 2340
actgttgtta gacgacggga ccgaggcagg tcccctagaa gaagaactcc ctcgcctcgc 2400
agacgcagat ctccatcgcc gcgtcgcaga agatctcaat ctcgggaatc tcaatgttag 2460
tattccttgg actcataagg tgggaaactt tacggggctt tattcctcta cagtacctat 2520
ctttaatcct gaatggcaaa ctccttcctt tcctaagatt catttacaag aggacattat 2580
taataggtgt caacaatttg tgggccctct cactgtaaat gaaaagagaa gattgaaatt 2640
aattatgcct gctagattct atcctaccca cactaaatat ttgcccttag acaaaggaat 2700
taaaccttat tatccagatc aggtagttaa tcattacttc caaaccagac attatttaca 2760
tactctttgg aaggctggta ttctatataa gcgggaaacc acacgtagcg catcattttg 2820
cgggtcacca tattcttggg aacaagagct acagcatggg aggttggtca tcaaaacctc 2880
gcaaaggcat ggggacgaat ctttctgttc ccaatcctct gggattcttt cccgatcatc 2940
agttggaccc tgcattcgga gccaactcaa acaatccaga ttgggacttc aaccccgtca 3000
aggacgactg gccagcagcc aaccaagtag gagtgggagc attcgggcca aggctcaccc 3060
ctccacacgg cggtattttg gggtggagcc ctcaggctca gggcatattg accacagtgt 3120

caacaattcc tcctcctgcc tccaccaatc ggcagtcagg aaggcagcct actcccatct 3180
ctccacctct aagagacagt catcctcagg ccatgcagtg g 3221